# FASCICULE DE BREVET EUROPEEN

(11) **EP 1 409 672 B1**
(45) Date de publication et mention de la délivrance du brevet: **08.07.2009**
(21) Numéro de dépôt: 02751246.6
(22) Date de dépôt: 14.06.2002
(51) Int. Cl.: C12N 15/11, A61K 31/7088, A61P 9/00

(54) **OLIGONUCLEOTIDES ANTISENS CAPABLES D'INHIBER LA FORMATION DES TUBES CAPILLAIRES**
ANTISENS-OLIGONUKLEOTIDE DIE FÄHIG SIND DIE HERSTELLUNG VON KAPILLARRÖHRCHEN ZU HEMMEN
ANTISENSE OLIGONUCLEOTIDES WHICH CAN INHIBIT THE FORMATION OF CAPILLARY TUBES BY ENDOTHELIAL CELLS

(30) Priorité: 14.06.2001 FR 0107805
(43) Date de publication de la demande: 21.04.2004
(73) Titulaire: GENE SIGNAL INTERNATIONAL SA, Epalinges, VD (CH)
(72) Inventeur: AL-MAHMOOD, Salman, 75014 Paris (FR)
(74) Mandataire: Nargolwalla, Cyra
(86) Numéro de dépôt international: PCT/FR2002/002067
(87) Numéro de publication internationale: WO 2002/103014

(56) Documents cités:
- EP-A- 1 010 433
- WO-A-92/13083
- WO-A-96/35791
- WALLACE WILLIAM C ET AL: "Amyloid precursor protein requires the insulin signaling pathway for neurotrophic activity." MOLECULAR BRAIN RESEARCH, vol. 52, no. 2, 15 décembre 1997 (1997-12-15), pages 213-227, XP001053575 ISSN: 0169-328X
- SURMACZ W ET AL: "OVEREXPRESSION OF INSULIN RECEPTON SUBSTRATE 1 (IRS-1) IN THE HUMAN BREAST CANCER CELL LINE MCF-7 INDUCES LOSS OF AND TRANSFORMATION 1" CLINICAL CANCER RESEARCH, vol. 1, no. 11, novembre 1995 (1995-11), pages 1429-1436, XP000560324 ISSN: 1078-0432
- D'AMBROSIO C ET AL: "TRANSFORMING POTENTIAL OF THE INSULIN RECEPTOR SUBSTRATE 1" CELL GROWTH AND DIFFERENTIATION, vol. 6, no. 5, 1 mai 1995 (1995-05-01), pages 557-562, XP000560325

### LISTAGE DE SEQUENCES

<110> AL MAHMOOD, SALMAN
<120> Oligonucleotides antisens capables d'inhiber la formation de tubes capillaires par les cellules endothéliales
<130> B6531-PCT-Juin 2002
<140> PCT/FR02/02067
   <141> 2002-06-14
<150> FR01/07805
   <151> 2001-06-14
<160> 29
<170> PatentIn version 3.1
<210> 1
   <211> 48
   <212> DNA
   <213> Séquence artificielle,
<220>
   <221> misc_feature
   <222> (1)..(48)
   <223> oligonucleotide sens
<400> 1
   tcgatgtgac gctactgatg agtccgtgag gacgaaactc tggcctag 48
<210> 2
   <211> 35
   <212> DNA
   <213> Séquence artificielle
<220>
   <221> misc_feature
   <222> (1)..(35)
   <223> Oligonucleotide anti-sens.
<400> 2
   tatccggagg gctcgccatg ctgctgcgga gcaga 35
<210> 3
   <211> 25
   <212> DNA
   <213> Séquence artificielle
<220>
   <221> misc_feature
   <222> (1)..(25)
   <223> Oligonucleotide anti-sens.
<400> 3
   tatccggagg gctcgccatg ctgct 25
<210> 4
   <211> 23
   <212> DNA
   <213> Séquence artificielle
<220>
   <221> misc_feature
   <222> (1)..(23)
   <223> Oligonucleotide anti-sens.
<400> 4
   tcgccatgct gctgcggagc aga 23
<210> 5
   <211> 25
   <212> DNA
   <213> Séquence artificielle
<220>
   <221> misc_feature
   <222> (1)..(25)
   <223> Oligonucleotide anti-sens.
<400> 5
   tatccggagg gcctgccatg ctgct 25
<210> 6
   <211> 24
   <212> DNA
   <213> Séquence artificielle
<220>
   <221> misc_feature
   <222> (1)..(24)
   <223> Oligonucleotide anti-sens.
<400> 6
   tatccggagg gcctgccatg ctgc 24
<210> 7
   <211> 23
   <212> DNA
   <213> Séquence artificielle
<220>
   <221> misc_feature
   <222> (1)..(23)
   <223> Oligonucleotide anti-sens.
<400> 7
   tatccggagg gcctgccatg ctg 23
<210> 8
   <211> 22
   <212> DNA
   <213> Séquence artificielle
<220>
   <221> misc_feature
   <222> (1)..(22)
   <223> Oligonucleotide anti-sens.
<400> 8
   tatccggagg gcctgccatg ct 22
<210> 9
   <211> 21
   <212> DNA
   <213> Séquence artificielle
<220>
   <221> misc_feature
   <222> (1)..(21)
   <223> Oligonucleotide anti-sens.
<400> 9
   tatccggagg gcctgccatg c 21
<210> 10
   <211> 20
   <212> DNA
   <213> Séquence artificielle
<220>
   <221> misc_feature
   <222> (1)..(20)
   <223> Oligonucleotide anti-sens.
<400> 10
   tatccggagg gcctgccatg 20
<210> 11
   <211> 19
   <212> DNA
   <213> Séquence artificielle
<220>
   <221> misc_feature
   <222> (1)..(19)
   <223> Oligonucleotide anti-sens.
<400> 11
   tatccggagg gcctgccat 19
<210> 12
   <211> 18
   <212> DNA
   <213> Séquence artificielle
<220>
   <221> misc_feature
   <222> (1)..(18)
   <223> Oligonucleotide anti-sens.
<400> 12
   tatccggagg gcctgcca 18
<210> 13
   <211> 17
   <212> DNA
   <213> Séquence artificielle
<220>
   <221> misc_feature
   <222> (1)..(17)
   <223> Oligonucleotide anti-sens.
<400> 13 17
   tatccggagg gcctgcc 17
<210> 14
   <211> 16
   <212> DNA
   <213> Séquence artificielle
<220>
   <221> misc_feature
   <222> (1)..(16)
   <223> Oligonucleotide anti-sens.
<400> 14
   tatccggagg gcctgc 16
<210> 15
   <211> 15
   <212> DNA
   <213> Séquence artificielle
<220>
   <221> misc_feature
   <222> (1).. (15)
   <223> Oligonucleotide anti-sens.
<400> 15
   tatccggagg gcctg 15
<210> 16
   <211> 14
   <212> DNA
   <213> Séquence artificielle
<220>
   <221> misc_feature
   <222> (1).. (14)
   <223> Oligonucleotide anti-sens.
<400> 16
   tatccggagg gcct 14
<210> 17
   <211> 13
   <212> DNA
   <213> Séquence artificielle
<220>
   <221> misc_feature
   <222> (1)..(13)
   <223> Oligonucleotide anti-sens.
<400> 17
   tatccggagg gcc 13
<210> 18
   <211> 12
   <212> DNA
   <213> Séquence artificielle
<220>
   <221> misc_feature
   <222> (1)..(12)
   <223> Ologonucleotide anti-sens.
<400> 18
   tatccggagg gc 12
<210> 19
   <211> 22
   <212> DNA
   <213> Séquence artificielle
<220>
   <221> misc_feature
   <222> (1)..(22)
   <223> Oligonucleotide anti-sens.
<400> 19
   ccggagggcc tgccatgctg ct 22
<210> 20
   <211> 19
   <212> DNA
   <213> Séquence artificielle
<220>
   <221> misc_feature
   <222> (1)..(19)
   <223> Oigonucleotide anti-sens.
<400> 20
   gagggcctgc catgctgct 19
<210> 21
   <211> 16
   <212> DNA
   <213> Séquence artificielle
<220>
   <221> misc_feature
   <222> (1)..(16)
   <223> Oligonucleotide anti-sens.
<400> 21
   ggcctgccat gctgct 16
<210> 22
   <211> 13
   <212> DNA
   <213> Séquence artificielle
<220>
   <221> misc_feature
   <222> (1)..(13)
   <223> Oligonucleotide anti-sens.
<400> 22
   ctgccatgct gct 13
<210> 23
   <211> 12
   <212> DNA
   <213> Séquence artificielle
<220>
   <221> misc_feature
   <222> (1)..(12)
   <223>. Oligonucleotide anti-sens.
<400> 23
   tgccatgctg ct 12
<210> 24
   <211> 14
   <212> DNA
   <213> Séquence artificielle
<220>
   <221> misc_feature
   <222> (1)..(14)
   <223> Oligonucleotide anti-sens;
<400> 24
   tttttttttt ttgg 14
<210> 25
   <211> 14
   <212> DNA
   <213> Séquence artificielle.
<220>
   <221> misc_feature
   <222> (1)..(14)
   <223> Oligonucleotide anti-sens.
<400> 25
   tttttttttt ttag 14
<210> 26
   <211> 14
   <212> DNA
   <213> Séquence artificielle
<220>
   <221> misc_feature
   <222> (1)..(14)
   <223> Oligonucleotide anti-sens.
<400> 26
   tttttttttt ttcg 14
<210> 27
   <211> 326
   <212> DNA
   <213> Homo sapiens
<220>
   <221> misc_feature
   <222> (1)..(326)
   <223> Fragment du gène IRS-1 codant pour le substrat du récepteur de 1' insuline.
<400> 27
   gtgccgagct gagttcctta taagaattaa tcttaatttt gtattttttc ctgtaagaca 60
   ataggccatg ttaattaaac tgaagaagga tatatttggc tgggtgtttt caaatgtcag 120
   cttaaaattg gtaattgaat ggaagcaaaa ttataagaag aggaaattaa agtcttccat 180
   tgcatgtatt gtaaacagaa ggagatgggt gattccttca attcaaaagc tctctttgga 240
   atgaacaatg tgggcgtttg taaattctgg aaatgtcttt ctattcataa taaactagat 300
   actgttgatc ttttaaaaaa aaaaaa 326
<210> 28
   <211> 5800
   <212> DNA
   <213> Homo sapiens
<220>
   <221> CDS
   <222> (1022)..(4750)
   <223>
<400> 28
   cggcggcgcg gtcggagggg gccggcgcgc agagccagac gccgccgctt gttttggttg 60
   gggctctcgg caactctccg aggaggagga ggaggaggga ggaggggaga agtaactgca    120
   gcggcagcgc ctcccgagga acaggcgtct tccccgaacc cttcccaaac ctcccccatc    180
   ccctctcgcc cttgtcccct cccctcctcc ccagccgcct ggagcgaggg gcagggatga    240
   gtctgtccct ccggccggtc cccagctgca gtggctgccc ggtatcgttt cgcatggaaa    300
   agccactttc tccacccgcc gagatgggcc cggatggggg ctgcagagga cgcgcccgcg    360
   ggcggcggca gcagcagcag cagcagcagc agcaacagca acagccgcag cgccgcggtc    420
   tctgcgactg agctggtatt tgggcggctg gtggcggctg ggacggttgg ggggtgggag    480
   gaggcgaagg aggagggaga accccgtgca acgttgggac ttggcaaccc gcctccccct   540
   gcccaaggat atttaatttg cctcgggaat cgctgcttcc agaggggaac tcaggaggga    600
   aggcgcgcgc gcgcgcgcgc tcctggaggg gcaccgcagg gacccccgac tgtcgcctcc    660
   ctgtgccgga ctccagccgg ggcgacgaga gatgcatctt cgctccttcc tggtggcggc    720
   ggcggctgag aggagacttg gctctcggag gatcggggct gccctcaccc cggacgcact    780
   gcctccccgc cgggcgtgaa gcgcccgaaa actccggtcg ggctctctcc tgggctcagc    840
   agctgcgtcc tccttcagct gcccctcccc ggcgcggggg gcggcgtgga tttcagagtc    900
   ggggtttctg ctgcctccag ccctgtttgc atgtgccggg ccgcggcgag gagcctccgc    960
   cccccacccg gttgtttttc ggagcctccc tctgctcagc gttggtggtg gcggtggcag    1020
   c atg gcg agc cct ccg gag agc gat ggc ttc tcg gac gtg cgc aag gtg    1069
     Met Ala Ser Pro Pro Glu Ser Asp Gly Phe Ser Asp Val Arg Lys Val
     1               5                   10                  15
   ggc tac ctg cgc aaa ccc aag agc atg cac aaa cgc ttc ttc gta ctg      1117
   Gly Tyr Leu Arg Lys Pro Lys Ser Met His Lys Arg Phe Phe Val Leu
               20                  25                  30
   cgc gcg gcc agc gag gct ggg ggc ccg gcg cgc ctc gag tac tac gag      1165
   Arg Ala Ala Ser Glu Ala Gly Gly Pro Ala Arg Leu Glu Tyr Tyr Glu
           35                  40                  45
   aac gag aag aag tgg cgg cac aag tcg agc gcc ccc aaa cgc tcg atc      1213
   Asn Glu Lys Lys Trp Arg His Lys Ser Ser Ala Pro Lys Arg Ser Ile
       50                  55                  60
   ccc ctt gag agc tgc ttc aac atc aac aag cgg gct gac tcc aag aac      1261
   Pro Leu Glu Ser Cys Phe Asn Ile Asn Lys Arg Ala Asp Ser Lys Asn
   65                  70                  75                  80
   aag cac ctg gtg gct ctc tac acc cgg gac gag cac ttt gcc atc gcg      1309
   Lys His Leu Val Ala Leu Tyr Thr Arg Asp Glu His Phe Ala Ile Ala
                   85                  90                  95
   gcg gac agc gag gcc gag caa gac agc tgg tac cag gct ctc cta cag      1357
   Ala Asp Ser Glu Ala Glu Gln Asp Ser Trp Tyr Gln Ala Leu Leu Gln
               100                 105                 110
   ctg cac aac cgt gct aag ggc cac cac gac gga gct gcg gcc ctc ggg      1405
   Leu His Asn Arg Ala Lys Gly His His Asp Gly Ala Ala Ala Leu Gly
           115                 120                 125
   gcg gga ggt ggt ggg ggc agc tgc agc ggc agc tcc ggc ctt ggt gag 1453
   Ala Gly Gly Gly Gly Gly Ser Cys Ser Gly Ser Ser Gly Leu Gly Glu
   130 135 140
   gct ggg gag gac ttg agc tac ggt gac gtg ccc cca gga ccc gca ttc 1501
   Ala Gly Glu Asp Leu Ser Tyr Gly Asp Val Pro Pro Gly Pro Ala Phe
   145 150 155 160
   aaa gag gtc tgg caa gtg atc ctg aag ccc aag ggc ctg ggt cag aca 1549
   Lys Glu Val Trp Gln Val Ile Leu Lys Pro Lys Gly Leu Gly Gln Thr
   165 170 175
   aag aac ctg att ggt atc tac cgc ctt tgc ctg acc agc aag acc atc 1597
   Lys Asn Leu Ile Gly Ile Tyr Arg Leu Cys Leu Thr Ser Lys Thr Ile
   180 185 190
   agc ttc gtg aag ctg aac tcg gag gca gcg gcc gtg gtg ctg cag ctg 1645
   Ser Phe Val Lys Leu Asn Ser Glu Ala Ala Ala Val Val Leu Gln Leu
   195 200 205
   atg aac atc agg cgc tgt ggc cac tcg gaa aac ttc ttc ttc atc gag 1693
   Met Asn Ile Arg Arg Cys Gly His Ser Glu Asn Phe Phe Phe Ile Glu
   210 215 220
   gtg ggc cgt tct gcc gtg acg ggg ccc ggg gag ttc tgg atg cag gtg 1741
   Val Gly Arg Ser Ala Val Thr Gly Pro Gly Glu Phe Trp Met Gln Val
   225 230 235 240
   gat gac tct gtg gtg gcc cag aac atg cac gag acc atc ctg gag gcc 1789
   Asp Asp Ser Val Val Ala Gln Asn Met His Glu Thr Ile Leu Glu Ala
   245 250 255
   atg cgg gcc atg agt gat gag ttc cgc cct cgc agc aag agc cag tcc 1837
   Met Arg Ala Met Ser Asp Glu Phe Arg Pro Arg Ser Lys Ser Gln Ser
   260 265 270
   tcg tcc aac tgc tct aac ccc atc agc gtc ccc ctg cgc cgg cac cat 1885
   Ser Ser Asn Cys Ser Asn Pro Ile Ser Val Pro Leu Arg Arg His His
   275 280 285
   ctc aac aat ccc ccg ccc agc cag gtg ggg ctg acc cgc cga tca cgc 1933
   Leu Asn Asn Pro Pro Pro Ser Gln Val Gly Leu Thr Arg Arg Ser Arg
   290 295 300
   act gag agc atc acc gcc acc tcc ccg gcc agc atg gtg ggc ggg aag 1981
   Thr Glu Ser Ile Thr Ala Thr Ser Pro Ala Ser Met Val Gly Gly Lys
   305 310 315 320
   cca ggc tcc ttc cgt gtc cgc gcc tcc agt gac ggc gaa ggc acc atg 2029
   Pro Gly Ser Phe Arg Val Arg Ala Ser Ser Asp Gly Glu Gly Thr Met
   325 330 335
   tcc cgc cca gcc tcg gtg gac ggc agc cct gtg agt ccc agc acc aac 2077
   Ser Arg Pro Ala Ser Val Asp Gly Ser Pro Val Ser Pro Ser Thr Asn
   340 345 350
   aga acc cac gcc cac cgg cat cgg ggc agc gcc cgg ctg cac ccc ccg 2125
   Arg Thr His Ala His Arg His Arg Gly Ser Ala Arg Leu His Pro Pro
   355 360 365
   ctc aac cac agc cgc tcc atc ccc atg ccg gct tcc cgc tgc tcg cct 2173
   Leu Asn His Ser Arg Ser Ile Pro Met Pro Ala Ser Arg Cys Ser Pro
           370                 375                 380
   tcg gcc acc agc ccg gtc agt ctg tcg tcc agt agc acc agt ggc cat      2221
   Ser Ala Thr Ser Pro Val Ser Leu Ser Ser Ser Ser Thr Ser Gly His
   385                 390                 395                 400
   ggc tcc acc tcg gat tgt ctc ttc cca cgg cga tct agt gct tcg gtg      2269
   Gly Ser Thr Ser Asp Cys Leu Phe Pro Arg Arg Ser Ser Ala Ser Val
                   405                 410                 415
   tct ggt tcc ccc agc gat ggc ggt ttc atc tcc tcg gat gag tat ggc      2317
   Ser Gly Ser Pro Ser Asp Gly Gly Phe Ile Ser Ser Asp Glu Tyr Gly
               420                 425                 430
   tcc agt ccc tgc gat ttc cgg agt tcc ttc cgc agt gtc act ccg gat      2365
   Ser Ser Pro Cys Asp Phe Arg Ser Ser Phe Arg Ser Val Thr Pro Asp
           435                 440                 445
   tcc ctg ggc cac acc cca cca gcc cgc ggt gag gag gag cta agc aac      2413
   Ser Leu Gly His Thr Pro Pro Ala Arg Gly Glu Glu Glu Leu Ser Asn
       450                 455                 460
   tat atc tgc atg ggt ggc aag ggg ccc tcc acc ctg acc gcc ccc aac      2461
   Tyr Ile Cys Met Gly Gly Lys Gly Pro Ser Thr Leu Thr Ala Pro Asn
   465                 470                 475                 480
   ggt cac tac att ttg tct cgg ggt ggc aat ggc cac cgc tgc acc cca      2509
   Gly His Tyr Ile Leu Ser Arg Gly Gly Asn Gly His Arg Cys Thr Pro
                   485                 490                 495
   gga aca ggc ttg ggc acg agt cca gcc ttg gct ggg gat gaa gca gcc      2557
   Gly Thr Gly Leu Gly Thr Ser Pro Ala Leu Ala Gly Asp Glu Ala Ala
               500                 505                 510
   agt gct gca gat ctg gat aat cgg ttc cga aag aga act cac tcg gca      2605
   Ser Ala Ala Asp Leu Asp Asn Arg Phe Arg Lys Arg Thr His Ser Ala
           515                 520                 525
   ggc aca tcc cct acc att acc cac cag aag acc ccg tcc cag tcc tca      2653
   Gly Thr Ser Pro Thr Ile Thr His Gln Lys Thr Pro Ser Gln Ser Ser
       530                 535                 540
   gtg gct tcc att gag gag tac aca gag atg atg cct gcc tac cca cca      2701
   Val Ala Ser Ile Glu Glu Tyr Thr Glu Met Met Pro Ala Tyr Pro Pro
   545                 550                 555                 560
   gga ggt ggc agt gga ggc cga ctg ccg gga cac agg cac tcc gcc ttc      2749
   Gly Gly Gly Ser Gly Gly Arg Leu Pro Gly His Arg His Ser Ala Phe
                   565                 570                 575
   gtg ccc acc cgc tcc tac cca gag gag ggt ctg gaa atg cac ccc ttg      2797
   Val Pro Thr Arg Ser Tyr Pro Glu Glu Gly Leu Glu Met His Pro Leu
               580                 585                 590
   gag cgt cgg ggg ggg cac cac cgc cca gac agc tcc acc ctc cac acg      2845
   Glu Arg Arg Gly Gly His His Arg Pro Asp Ser Ser Thr Leu His Thr
           595                 600                 605
   gat gat ggc tac atg ccc atg tcc cca ggg gtg gcc cca gtg ccc agt      2893
   Asp Asp Gly Tyr Met Pro Met Ser Pro Gly Val Ala Pro Val Pro Ser
       610                 615                 620
   ggc cga aag ggc agt gga gac tat atg ccc atg agc ccc aag agc gta      2941
   Gly Arg Lys Gly Ser Gly Asp Tyr Met Pro Met Ser Pro Lys Ser Val
   625                 630                 635                 640
   tct gcc cca cag cag atc atc aat ccc atc aga cgc cat ccc cag aga      2989
   Ser Ala Pro Gln Gln Ile Ile Asn Pro Ile Arg Arg His Pro Gln Arg
                   645                 650                 655
   gtg gac ccc aat ggc tac atg atg atg tcc ccc agc ggt ggc tgc tct      3037
   Val Asp Pro Asn Gly Tyr Met Met Met Ser Pro Ser Gly Gly Cys Ser
               660                 665                 670
   cct gac att gga ggt ggc ccc agc agc agc agc agc agc agc aac gcc      3085
   Pro Asp Ile Gly Gly Gly Pro Ser Ser Ser Ser Ser Ser Ser Asn Ala
           675                 680                 685
   gtc cct tcc ggg acc agc tat gga aag ctg tgg aca aac ggg gta ggg      3133
   Val Pro Ser Gly Thr Ser Tyr Gly Lys Leu Trp Thr Asn Gly Val Gly
       690                 695                 700
   ggc cac cac tct cat gtc ttg cct cac ccc aaa ccc cca gtg gag agc      3181
   Gly His His Ser His Val Leu Pro His Pro Lys Pro Pro Val Glu Ser
   705                 710                 715                 720
   agc ggt ggt aag ctc tta cct tgc aca ggt gac tac atg aac atg tca      3229
   Ser Gly Gly Lys Leu Leu Pro Cys Thr Gly Asp Tyr Met Asn Met Ser
                   725                 730                 735
   cca gtg ggg gac tcc aac acc agc agc ccc tcc gac tgc tac tac ggc      3277
   Pro Val Gly Asp Ser Asn Thr Ser Ser Pro Ser Asp Cys Tyr Tyr Gly
               740                 745                 750
   cct gag gac ccc cag cac aag cca gtc ctc tcc tac tac tca ttg cca      3325
   Pro Glu Asp Pro Gln His Lys Pro Val Leu Ser Tyr Tyr Ser Leu Pro
           755                 760                 765
   aga tcc ttt aag cac acc cag cgc ccc ggg gag ccg gag gag ggt gcc      3373
   Arg Ser Phe Lys His Thr Gln Arg Pro Gly Glu Pro Glu Glu Gly Ala
       770                 775                 780
   cgg cat cag cac ctc cgc ctt tcc act agc tct ggt cgc ctt ctc tat      3421
   Arg His Gln His Leu Arg Leu Ser Thr Ser Ser Gly Arg Leu Leu Tyr
   785                 790                 795                 800
   gct gca aca gca gat gat tct tcc tct tcc acc agc agc gac agc ctg      3469
   Ala Ala Thr Ala Asp Asp Ser Ser Ser Ser Thr Ser Ser Asp Ser Leu
                   805                 810                 815
   ggt ggg gga tac tgc ggg gct agg ctg gag ccc agc ctt cca cat ccc      3517
   Gly Gly Gly Tyr Cys Gly Ala Arg Leu Glu Pro Ser Leu Pro His Pro
               820                 825                 830
   cac cat cag gtt ctg cag ccc cat ctg cct cga aag gtg gac aca gct      3565
   His His Gln Val Leu Gln Pro His Leu Pro Arg Lys Val Asp Thr Ala
           835                 840                 845
   gct cag acc aat agc cgc ctg gcc cgg ccc acg agg ctg tcc ctg ggg      3613
   Ala Gln Thr Asn Ser Arg Leu Ala Arg Pro Thr Arg Leu Ser Leu Gly
       850                 855                 860
   gat ccc aag gcc agc acc tta cct cgg gcc cga gag cag cag cag cag      3661
   Asp Pro Lys Ala Ser Thr Leu Pro Arg Ala Arg Glu Gln Gln Gln Gln
   865                 870                 875                 880
   cag cag ccc ttg ctg cac cct cca gag ccc aag agc ccg ggg gaa tat 3709
   Gln Gln Pro Leu Leu His Pro Pro Glu Pro Lys Ser Pro Gly Glu Tyr
   885 890 895
   gtc aat att gaa ttt ggg agt gat cag tct ggc tac ttg tct ggc ccg 3757
   Val Asn Ile Glu Phe Gly Ser Asp Gln Ser Gly Tyr Leu Ser Gly Pro
   900 905 910
   gtg gct ttc cac agc tca cct tct gtc agg tgt cca tcc cag ctc cag 3805
   Val Ala Phe His Ser Ser Pro Ser Val Arg Cys Pro Ser Gln Leu Gln
   915 920 925
   cca gct ccc aga gag gaa gag act ggc act gag gag tac atg aag atg 3853
   Pro Ala Pro Arg Glu Glu Glu Thr Gly Thr Glu Glu Tyr Met Lys Met
   930 935 940
   gac ctg ggg ccg ggc cgg agg gca gcc tgg cag gag agc act ggg gtc 3901
   Asp Leu Gly Pro Gly Arg Arg Ala Ala Trp Gln Glu Ser Thr Gly Val
   945 950 955 960
   gag atg ggc aga ctg ggc cct gca cct ccc ggg gct gct agc att tgc 3949
   Glu Met Gly Arg Leu Gly Pro Ala Pro Pro Gly Ala Ala Ser Ile Cys
   965 970 975
   agg cct acc cgg gca gtg ccc agc agc cgg ggt gac tac atg acc atg 3997
   Arg Pro Thr Arg Ala Val Pro Ser Ser Arg Gly Asp Tyr Met Thr Met
   980 985 990
   cag atg agt tgt ccc cgt cag agc tac gtg gac acc tcg cca gct gcc 4045
   Gln Met Ser Cys Pro Arg Gln Ser Tyr Val Asp Thr Ser Pro Ala Ala
   995 1000 1005
   cct gta agc tat gct gac atg cga aca ggc att gct gca gag gag 4090
   Pro Val Ser Tyr Ala Asp Met Arg Thr Gly Ile Ala Ala Glu Glu
   1010 1015 1020
   gtg agc ctg ccc agg gcc acc atg gct gct gcc tcc tca tcc tca 4135
   Val Ser Leu Pro Arg Ala Thr Met Ala Ala Ala Ser Ser Ser Ser
   1025 1030 1035
   gca gcc tct gct tcc ccg act ggg cct caa ggg gca gca gag ctg 4180
   Ala Ala Ser Ala Ser Pro Thr Gly Pro Gln Gly Ala Ala Glu Leu
   1040 1045 1050
   gct gcc cac tcg tcc ctg ctg ggg ggc cca caa gga cct ggg ggc 4225
   Ala Ala His Ser Ser Leu Leu Gly Gly Pro Gln Gly Pro Gly Gly
   1055 1060 1065
   atg agc gcc ttc acc cgg gtg aac ctc agt cct aac cgc aac cag 4270
   Met Ser Ala Phe Thr Arg Val Asn Leu Ser Pro Asn Arg Asn Gln
   1070 1075 1080
   agt gcc aaa gtg atc cgt gca gac cca caa ggg tgc cgg cgg agg 4315
   Ser Ala Lys Val Ile Arg Ala Asp Pro Gln Gly Cys Arg Arg Arg
   1085 1090 1095
   cat agc tcc gag act ttc tcc tca aca ccc agt gcc acc cgg gtg 4360
   His Ser Ser Glu Thr Phe Ser Ser Thr Pro Ser Ala Thr Arg Val
   1100 1105 1110
   ggc aac aca gtg ccc ttt gga gcg ggg gca gca gta ggg ggc ggt 4405
   Gly Asn Thr Val Pro Phe Gly Ala Gly Ala Ala Val Gly Gly Gly
   1115 1120 1125
   ggc ggt agc agc agc agc agc gag gat gtg aaa cgc cac agc tct      4450
   Gly Gly Ser Ser Ser Ser Ser Glu Asp Val Lys Arg His Ser Ser
       1130                1135                1140
   gct tcc ttt gag aat gtg tgg ctg agg cct ggg gag ctt ggg gga      4495
   Ala Ser Phe Glu Asn Val Trp Leu Arg Pro Gly Glu Leu Gly Gly
       1145                1150                1155
   gcc ccc aag gag cca gcc aaa ctg tgt ggg gct gct ggg ggt ttg      4540
   Ala Pro Lys Glu Pro Ala Lys Leu Cys Gly Ala Ala Gly Gly Leu
       1160                1165                1170
   gag aat ggt ctt aac tac ata gac ctg gat ttg gtc aag gac ttc      4585
   Glu Asn Gly Leu Asn Tyr Ile Asp Leu Asp Leu Val Lys Asp Phe
       1175                1180                1185
   aaa cag tgc cct cag gag tgc acc cct gaa ccg cag cct ccc cca      4630
   Lys Gln Cys Pro Gln Glu Cys Thr Pro Glu Pro Gln Pro Pro Pro
       1190                1195                1200
   ccc cca ccc cct cat caa ccc ctg ggc agc ggt gag agc agc tcc      4675
   Pro Pro Pro Pro His Gln Pro Leu Gly Ser Gly Glu Ser Ser Ser
       1205                1210                1215
   acc cgc cgc tca agt gag gat tta agc gcc tat gcc agc atc agt      4720
   Thr Arg Arg Ser Ser Glu Asp Leu Ser Ala Tyr Ala Ser Ile Ser
       1220                1225                1230
   ttc cag aag cag cca gag gac cgt cag tag ctcaactgga catcacagca    4770
   Phe Gln Lys Gln Pro Glu Asp Arg Gln
       1235                1240
   gaatgaagac ctaaatgacc tcagcaaatc ctcttctaac tcatgggtac ccagactcta 4830
   aatatttcat gattcacaac taggacctca tatcttcctc atcagtagat ggtacgatgc 4890
   atccatttca gtttgtttac tttatccaat cctcaggatt tcattgactg aactgcacgt 4950
   tctatattgt gccaagcgaa aaaaaaaaat gcactgtgac accagaataa tgagtctgca 5010
   taaacttcat cttcaacctt aaggacttag ctggccacag tgagctgatg tgcccaccac 5070
   cgtgtcatga gagaatgggt ttactctcaa tgcattttca agatacattt catctgctgc 5130
   tgaaactgtg tacgacaaag catcattgta aattatttca tacaaaactg ttcacgttgg 5190
   gtggagagag tattaaatat ttaacatagg ttttgattta tatgtgtaat tttttaaatg 5250
   aaaatgtaac ttttcttaca gcacatcttt tttttggatg tgggatggag gtatacaatg 5310
   ttctgttgta aagagtggag caaatgctta aaacaaggct taaaagagta gaatagggta 5370
   tgatccttgt tttaagattg taattcagaa aacataatat aagaatcata gtgccataga 5430
   tggttctcaa ttgtatagtt atatttgctg atactatctc ttgtcatata aacctgatgt 5490
   tgagctgagt tccttataag aattaatctt aattttgtat tttttcctgt aagacaatag 5550
   gccatgttaa ttaaactgaa gaaggatata tttggctggg tgttttcaaa tgtcagctta 5610
   aaattggtaa ttgaatggaa gcaaaattat aagaagagga aattaaagtc ttccattgca 5670
   tgtattgtaa acagaaggag atgggtgatt ccttcaattc aaaagctctc tttggaatga 5730
   acaatgtggg cgtttgtaaa ttctggaaat gtctttctat tcataataaa ctagatactg 5790
   ttgatctttt 5800
<210> 29
   <211> 1242
   <212> PRT
   <213> Homo sapiens
<400> 29
   Met Ala Ser Pro Pro Glu Ser Asp Gly Phe Ser Asp Val Arg Lys Val
   1 5 10 15
   Gly Tyr Leu Arg Lys Pro Lys Ser Met His Lys Arg Phe Phe Val Leu
   20 25 30
   Arg Ala Ala Ser Glu Ala Gly Gly Pro Ala Arg Leu Glu Tyr Tyr Glu
   35 40 45
   Asn Glu Lys Lys Trp Arg His Lys Ser Ser Ala Pro Lys Arg Ser Ile
   50 55 60
   Pro Leu Glu Ser Cys Phe Asn Ile Asn Lys Arg Ala Asp Ser Lys Asn
   65 70 75 80
   Lys His Leu Val Ala Leu Tyr Thr Arg Asp Glu His Phe Ala Ile Ala
   85 90 95
   Ala Asp Ser Glu Ala Glu Gln Asp Ser Trp Tyr Gln Ala Leu Leu Gln
   100 105 110
   Leu His Asn Arg Ala Lys Gly His His Asp Gly Ala Ala Ala Leu Gly
   115 120 125
   Ala Gly Gly Gly Gly Gly Ser Cys Ser Gly Ser Ser Gly Leu Gly Glu
   130 135 140
   Ala Gly Glu Asp Leu Ser Tyr Gly Asp Val Pro Pro Gly Pro Ala Phe
   145 150 155 160
   Lys Glu Val Trp Gln Val Ile Leu Lys Pro Lys Gly Leu Gly Gln Thr
   165 170 175
   Lys Asn Leu Ile Gly Ile Tyr Arg Leu Cys Leu Thr Ser Lys Thr Ile
   180 185 190
   Ser Phe Val Lys Leu Asn Ser Glu Ala Ala Ala Val Val Leu Gln Leu
   195 200 205
   Met Asn Ile Arg Arg Cys Gly His Ser Glu Asn Phe Phe Phe Ile Glu
   210 215 220
   Val Gly Arg Ser Ala Val Thr Gly Pro Gly Glu Phe Trp Met Gln Val
   225 230 235 240
   Asp Asp Ser Val Val Ala Gln Asn Met His Glu Thr Ile Leu Glu Ala
   245 250 255
   Met Arg Ala Met Ser Asp Glu Phe Arg Pro Arg Ser Lys Ser Gln Ser
   260 265 270
   Ser Ser Asn Cys Ser Asn Pro Ile Ser Val Pro Leu Arg Arg His His
   275 280 285
   Leu Asn Asn Pro Pro Pro Ser Gln Val Gly Leu Thr Arg Arg Ser Arg
   290 295 300
   Thr Glu Ser Ile Thr Ala Thr Ser Pro Ala Ser Met Val Gly Gly Lys
   305 310 315 320
   Pro Gly Ser Phe Arg Val Arg Ala Ser Ser Asp Gly Glu Gly Thr Met
   325 330 335
   Ser Arg Pro Ala Ser Val Asp Gly Ser Pro Val Ser Pro Ser Thr Asn
   340 345 350
   Arg Thr His Ala His Arg His Arg Gly Ser Ala Arg Leu His Pro Pro
   355 360 365
   Leu Asn His Ser Arg Ser Ile Pro Met Pro Ala Ser Arg Cys Ser Pro
   370 375 380
   Ser Ala Thr Ser Pro Val Ser Leu Ser Ser Ser Ser Thr Ser Gly His
   385 390 395 400
   Gly Ser Thr Ser Asp Cys Leu Phe Pro Arg Arg Ser Ser Ala Ser Val
   405 410 415
   Ser Gly Ser Pro Ser Asp Gly Gly Phe Ile Ser Ser Asp Glu Tyr Gly
   420 425 430
   Ser Ser Pro Cys Asp Phe Arg Ser Ser Phe Arg Ser Val Thr Pro Asp
   435 440 445
   Ser Leu Gly His Thr Pro Pro Ala Arg Gly Glu Glu Glu Leu Ser Asn
   450 455 460
   Tyr Ile Cys Met Gly Gly Lys Gly Pro Ser Thr Leu Thr Ala Pro Asn
   465 470 475 480
   Gly His Tyr Ile Leu Ser Arg Gly Gly Asn Gly His Arg Cys Thr Pro
   485 490 495
   Gly Thr Gly Leu Gly Thr Ser Pro Ala Leu Ala Gly Asp Glu Ala Ala
   500 505 510
   Ser Ala Ala Asp Leu Asp Asn Arg Phe Arg Lys Arg Thr His Ser Ala
   515 520 525
   Gly Thr Ser Pro Thr Ile Thr His Gln Lys Thr Pro Ser Gln Ser Ser
   530 535 540
   Val Ala Ser Ile Glu Glu Tyr Thr Glu Met Met Pro Ala Tyr Pro Pro
   545 550 555 560
   Gly Gly Gly Ser Gly Gly Arg Leu Pro Gly His Arg His Ser Ala Phe
   565 570 575
   Val Pro Thr Arg Ser Tyr Pro Glu Glu Gly Leu Glu Met His Pro Leu
   580 585 590
   Glu Arg Arg Gly Gly His His Arg Pro Asp Ser Ser Thr Leu His Thr
   595 600 605
   Asp Asp Gly Tyr Met Pro Met Ser Pro Gly Val Ala Pro Val Pro Ser
   610 615 620
   Gly Arg Lys Gly Ser Gly Asp Tyr Met Pro Met Ser Pro Lys Ser Val
   625 630 635 640
   Ser Ala Pro Gln Gln Ile Ile Asn Pro Ile Arg Arg His Pro Gln Arg
   645 650 655
   Val Asp Pro Asn Gly Tyr Met Met Met Ser Pro Ser Gly Gly Cys Ser
   660 665 670
   Pro Asp Ile Gly Gly Gly Pro Ser Ser Ser Ser Ser Ser Ser Asn Ala
   675 680 685
   Val Pro Ser Gly Thr Ser Tyr Gly Lys Leu Trp Thr Asn Gly Val Gly
   690 695 700
   Gly His His Ser His Val Leu Pro His Pro Lys Pro Pro Val Glu Ser
   705 710 715 720
   Ser Gly Gly Lys Leu Leu Pro Cys Thr Gly Asp Tyr Met Asn Met Ser
   725 730 735
   Pro Val Gly Asp Ser Asn Thr Ser Ser Pro Ser Asp Cys Tyr Tyr Gly
   740 745 750
   Pro Glu Asp Pro Gln His Lys Pro Val Leu Ser Tyr Tyr Ser Leu Pro
   755 760 765
   Arg Ser Phe Lys His Thr Gln Arg Pro Gly Glu Pro Glu Glu Gly Ala
   770 775 780
   Arg His Gln His Leu Arg Leu Ser Thr Ser Ser Gly Arg Leu Leu Tyr
   785 790 795 800
   Ala Ala Thr Ala Asp Asp Ser Ser Ser Ser Thr Ser Ser Asp Ser Leu
   805 810 815
   Gly Gly Gly Tyr Cys Gly Ala Arg Leu Glu Pro Ser Leu Pro His Pro
   820 825 830
   His His Gln Val Leu Gln Pro His Leu Pro Arg Lys Val Asp Thr Ala
   835 840 845
   Ala Gln Thr Asn Ser Arg Leu Ala Arg Pro Thr Arg Leu Ser Leu Gly
   850 855 860
   Asp Pro Lys Ala Ser Thr Leu Pro Arg Ala Arg Glu Gln Gln Gln Gln
   865 870 875 880
   Gln Gln Pro Leu Leu His Pro Pro Glu Pro Lys Ser Pro Gly Glu Tyr
   885 890 895
   Val Asn Ile Glu Phe Gly Ser Asp Gln Ser Gly Tyr Leu Ser Gly Pro
   900 905 910
   Val Ala Phe His Ser Ser Pro Ser Val Arg Cys Pro Ser Gln Leu Gln
   915 920 925
   Pro Ala Pro Arg Glu Glu Glu Thr Gly Thr Glu Glu Tyr Met Lys Met
   930 935 940
   Asp Leu Gly Pro Gly Arg Arg Ala Ala Trp Gln Glu Ser Thr Gly Val
   945 950 955 960
   Glu Met Gly Arg Leu Gly Pro Ala Pro Pro Gly Ala Ala Ser Ile Cys
   965 970 975
   Arg Pro Thr Arg Ala Val Pro Ser Ser Arg Gly Asp Tyr Met Thr Met
   980 985 990
   Gln Met Ser Cys Pro Arg Gln Ser Tyr Val Asp Thr Ser Pro Ala Ala
   995 1000 1005
   Pro Val Ser Tyr Ala Asp Met Arg Thr Gly Ile Ala Ala Glu Glu
   1010 1015 1020
   Val Ser Leu Pro Arg Ala Thr Met Ala Ala Ala Ser Ser Ser Ser
   1025 1030 1035
   Ala Ala Ser Ala Ser Pro Thr Gly Pro Gln Gly Ala Ala Glu Leu
   1040 1045 1050
   Ala Ala His Ser Ser Leu Leu Gly Gly Pro Gln Gly Pro Gly Gly
   1055 1060 1065
   Met Ser Ala Phe Thr Arg Val Asn Leu Ser Pro Asn Arg Asn Gln
   1070 1075 1080
   Ser Ala Lys Val Ile Arg Ala Asp Pro Gln Gly Cys Arg Arg Arg
   1085 1090 1095
   His Ser Ser Glu Thr Phe Ser Ser Thr Pro Ser Ala Thr Arg Val
   1100 1105 1110
   Gly Asn Thr Val Pro Phe Gly Ala Gly Ala Ala Val Gly Gly Gly
   1115 1120 1125
   Gly Gly Ser Ser Ser Ser Ser Glu Asp Val Lys Arg His Ser Ser
   1130 1135 1140
   Ala Ser Phe Glu Asn Val Trp Leu Arg Pro Gly Glu Leu Gly Gly
   1145 1150 1155
   Ala Pro Lys Glu Pro Ala Lys Leu Cys Gly Ala Ala Gly Gly Leu
   1160 1165 1170
   Glu Asn Gly Leu Asn Tyr Ile Asp Leu Asp Leu Val Lys Asp Phe
   1175 1180 1185
   Lys Gln Cys Pro Gln Glu Cys Thr Pro Glu Pro Gln Pro Pro Pro
   1190 1195 1200
   Pro Pro Pro Pro His Gln Pro Leu Gly Ser Gly Glu Ser Ser Ser
   1205 1210 1215
   Thr Arg Arg Ser Ser Glu Asp Leu Ser Ala Tyr Ala Ser Ile Ser
   1220 1225 1230
   Phe Gln Lys Gln Pro Glu Asp Arg Gln
   1235 1240

## Description

La présente invention a pour objet des oligonucléotides antisens capables d'inhiber l'expression de la protéine IRS-1 et d'inhiber la formation des tubes capillaires par les cellules endothéliales. Les oligonucléotides selon l'invention sont donc indiqués comme agent anti-angiogénique. Ils sont également indiqués comme agents anti-multiplication cellulaire, en particulier comme agenta anti-tumoraux.

L'invention concerne aussi des compositions pharmaceutiques contenant lesdits oligonucléotides et l'utilisation desdits oligonucléotides comme réactifs d'analyse.

L'angiogénèse est un processus fondamental par lequel de nouveaux vaisseaux sanguins se forment. Ce processus est essentiel dans plusieurs phénomènes physiologiques normaux tels que la reproduction, le développement ou encore la cicatrisation. Dans ces phénomènes biologiques normaux, l'angiogénèse est sous contrôle strict, c'est-à-dire qu'elle est déclenchée pendant une période courte (quelques jours), puis complètement inhibée. Cependant, plusieurs pathologies sont liées à une angiogénèse invasive et incontrôlée; l'arthrite, une pathologie due à l'endommagement des cartilages par les néovaisseaux invasifs; la rétinopathie diabétique où l'invasion de la rétine par les néovaisseaux conduit à l'aveuglement des malades; la néovascularisation de l'appareil oculaire présente la cause majeure de l'aveuglement, et cette néovascularisation intervient dans une vingtaine de maladies de l'oeil; ou encore la croissance et la métastase des tumeurs qui sont liées directement à la néovascularisation et sont dépendantes de l'angiogénèse. La tumeur stimule la croissance des néovaisseaux pour sa croissance elle-même. De plus, ces néovaisseaux sont des voies d'échappement des tumeurs qui joignent ainsi la circulation sanguine et provoquent des métastases dans des sites éloignés du foyer tumoral initial, comme le foie, le poumon ou l'os.

La formation de néovaisseaux par les cellules endothéliales, l'angiogénèse, implique la migration, la croissance, et la différentiation des cellules endothéliales. La régulation de ces phénomènes biologiques est directement liée à l'expression génétique.

Ainsi, les travaux de recherche réalisés dans le cadre de la présente invention ont permis d'identifier et de préparer des séquences d'acide nucléique impliquées dans la régulation de l'angiogénèse.

D'autres travaux concernant l'angiogénèse ont permis de mettre en évidence une expression importante et une phosphorylation, au niveau d'un résidu tyrosine d'une protéine, intracellulaire de 180 kDa, par les cellules endothéliales cultivées sur un tapis du collagène type **I** et stimulées par un facteur angiogénique tel que le bFGF. L'expression importante et la phosphorylation au niveau du résidu tyrosine de la protéine intracellulaire de 180 kDa accompagnent la formation des tubes capillaires par les cellules endothéliales.

Cette protéine est déjà connue en tant que substrat du récepteur de l'insuline (nommé IRS-1). Elle a été, en effet, partiellement identifiée et étudiée par certains auteurs travaillant sur le diabète (Quon et al., J. Biol. Chem. (1994), 269 (45), 27920-27924).

Ces auteurs ont étudié le rôle de l'IRS-1 sur (i) la translocation du GLUT 4 stimulée par l'insuline et (ii) le transport du glucose, dans des cellules adipeuses de rat. Pour ce faire, ils ont construit un plasmide contenant :
- un oligonucléotide bicaténaire, obtenu à partir de l'oligonucléotide sens de séquence SEQ ID No. 1 suivante : 5'-TCGATGTGAC GCTACTGATG AGTCCGTGAG GACGAAACTC TGGCCTAG-3', et
- l'ADNc codant pour l'IRS-1 humain,
   et ont transfecté des cellules adipeuses de rat avec ledit plasmide.

D'autre part, différents auteurs se sont intéressés à l'inhibition d'IRS-1 ; par exemple, WO9635791 décrit l'inhibition d'IRS-1 par des ribozymes et leur application comme agent anti-multiplication cellulaire, en particulier comme agent anti-tumoral. Toutefois, WO9635791 ne met pas à disposition une solution permettant d'inhiber efficacement l'expression de IRS-1, protéine impliquée dans un grand nombre de maladies liées à l'angiogénèse.

L'article MOL. BRAIN RES. 52(1997), 213-27 montre que la protéine précurseur d'amyloïde (APP) potentialise l'activité neurotrophique du facteur de croissance neural (NGF) vis-à-vis de la lignée cellulaire immortalisée PC12. Cette étude concerne donc la croissance des neurones et l'effet synergique de la protéine précurseur d'amyloïde (APP) et du facteur de croissance neural (NGF) sur l'état trophique neural. Elle montre également que l'incubation de cellules naïves avec des oligonucléotides antisens de IRS-1 conduit à l'inhibition de la croissance neurale stimulée par l'APP. Toutefois, les facteurs extracellulaires utilisés dans cet article (NGF et APP) n'ont aucun rapport avec l'angiogénèse.

L'article CLIN. CANCER RES. 1(1995, 1429-36 décrit l'action synergique de l'oestrogène et des facteurs de croissance analogues de l'insuline (IGFs) pour promouvoir la croissance de plusieurs lignées cellulaires de cancer du poumon. Cet article indique également que la surexpression d'IRS-1 entraîne une réponse cellulaire accrue à IGF-1, pour cette lignée de cellules du cancer du sein. Cette confirmation est apportée par l'application d'une stratégie antisens, dans laquelle il est démontré que l'utilisation d'un ARN antisens d'IRS-1 ou d'un ribosyme anti-IRS-1 inhibe la prolifération des cellules MCF-7 et leur dépendance à l'oestrogène.

Toutefois, aucun de ces documents ne décrit ni ne suggère qu'un oligonucléotide antisens d'IRS-1 serait utile en tant que principe actif dans une composition pharmaceutique destinées au traitement de maladies liées à l'angiogénèse.

Les travaux menés dans le cadre de la présente invention ont mis en évidence le fait que l'expression de la protéine IRS-1 est également induite dans les cellules endothéliales lorsque ces dernières sont stimulées par le facteur angiogénique bFGF.

L'invention concerne donc une composition pharmaceutique active sur les phénomènes d'angiogénèse comprenant à titre d'agent actif au moins une substance choisie parmi : (i) une molécule d'acide nucléique du gène codant pour la protéine IRS-1, d'une séquence complémentaire ou d'un fragment de celle-ci ou un fragment de celle-ci, (ii) une molécule capable d'inhiber l'expression d'une molécule d'acide nucléique selon (i).

Dans le cadre de l'invention, des oligonucléotides anti-sens du gène codant pour cette protéine ont été préparés. Ces oligonucléotides présentent des activités anti-angiogénique et anti-tumorale remarquables. Ils sont donc particulièrement utiles pour le traitement des maladies liées à une angiogénèse invasive et non contrôlée par des méthodes de thérapies géniques consistant à administrer à un individu une composition contenant au moins un de ces oligonucléotides.

Ainsi, un oligonucléotide selon l'invention est constitué par la séquence nucléôtidique de formule SEQ ID No.3 suivante :
5'-TATCCGGAGGGCTCGCCATGCTGCT-3',
   un fragment de celle-ci comprenant au moins 12 nucléotides contigus, ou leur dérivé.

On entend par dérivé, une séquence capable de s'hybrider dans des conditions strictes avec la séquence SEQ ID No. 3 ou avec un fragment de celles-ci d'au moins 12 nucléotides contigus.

Avantageusement, tout ou partie des liaisons phosphodiester des oligonucléotides de l'invention sont protégées. Cette protection s'effectue généralement par voie chimique, selon des méthodes classiques bien connues de l'homme du métier. Par exemple, on peut protéger les liaisons phosphodiester par une fonction thiol ou amine ou bien encore par un groupement phényle.

De façon également aventageuse, les extrémité 5'- et/ou 3'- des oligonucléotides de l'invention sont protégées, par exemple en utilisant la technique indiquée précédemment pour protéger les liaisons phosphodiester.

Les oligonucléotides de l'invention peuvent être synthétisés selon les techniques conventionnelles bien connues de l'homme du métier, par exemple à l'aide d'un synthétiseur d'ADN commercialisé par différentes sociétés.

Bien que leur mécanisme d'action ne soit pas entièrement élucidé, les oligonucléotides selon l'invention inhibent l'expression de la protéine IRS-1 au sein des cellules endothéliales. Ces oligonucléotides sont capables de bloquer la formation des néovaisseaux par les cellules endothéliales (i.e. inhibent l'angiogénèse), et de ce fait ils inhibent la multiplication des cellules tumorales chez la souris.

L'invention a donc aussi pour objet une composition pharmaceutique pour l'inhibition du gène codant pour la protéine IRS-1 comprenant au moins un oligonucléotide complémentaire d'une partie dudit gène ou d'un transcrit dudit gène.

De préférence, la molécule capable d'inhiber l'expression d'une molécule d'acide nucléique du gène codant pour la protéine IRS-1 est une séquence antisens de la région codante de la séquence identifiée sous le numéro SEQ ID No. 28 dans la liste de séquences en annexe.

Avantageusement, ladite séquence antisens comprend au moins douze nucléotides contigus ou leur dérivé.

Plus préférentiellement, l'agent actif capable d'inhiber l'expression d'une molécule d'acide nucléique du gène codant pour la protéine IRS-1 de la composition de l'invention est une séquence nucléotidique. identifiée sous le numéro SEQ ID No 3 comprenant au moins douze nucléotides contigus ou leur dérivé.

Une telle composition comprend avantageusement comme agent actif aux moins un oligonucléotide défini précédemment, avantageusement associé dans ladite composition avec un véhicule acceptable.

L'analyse des travaux réalisés dans le cadre de l'invention a donc permis de montrer que la protéine IRS-1 représente un constituant cellulaire essentiel dans le processus de l'angiogénèse. En effet, l'inhibition de l'expression de la protéine IRS-1 par lesdits oligonucléotides antisens conduit à l'inhibition de la formation de tubes capillaires par les cellules endothéliales.

Les oligonucléotides selon l'invention et les compositions les contenant sont donc indiqués comme agent anti-angiogénique. Ils sont également indiqués comme agents anti-multiplication cellulaire, en particulier comme agents anti-tumoraux, et en conséquence sont tout particulièrement utiles pour le traitement des tumeurs. L'invention a donc pour objet l'utilisation desdits oligonucléotides pour la préparation d'une composition destinée au traitement ou à la prévention de pathologies liées à une angiogénèse invasive et incontrôlée, comme par exemple à titre non limitatif: le traitement de la vascularisation de tumeurs, des maladies de l'oeil liées à la néovascularisation de l'appareil oculaire telles que les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose.

Les compositions pharmaceutiques ci-dessus sont plus particulièrement réalisées de manière à pouvoir être administrées par voie sous-cutanée, intramusculaire, intraveineuse ou transdermique. Pour une telle administration, on utilise notamment des suspensions aqueuses, des solutions salines isotoniques ou des solutions stériles et injectable qui contiennent des agents de dispersion et/ou des agents mouillants pharmacologiquement compatibles, par exemple le propylèneglycol ou le butylèneglycol.

La dose unitaire usuelle à administrer contient de 0,001 mg à 50 mg de principe actif.

Les oligonucléotides de l'invention sont aussi utiles comme réactif de recherche notamment pour l'étude *in vitro* des voies de signalisation impliquant la protéine 180 kDa, par exemple sur des cellules, tumorales ou non, transfectées par lesdits oligonucléotides. Ils sont également utiles pour l'étude *in vivo* des voies de signalisation impliquant la protéine 180 kDa dans un grand nombre de phénomènes physiologiques et pathologiques, comme l'angiogénèse ou la cancérogenèse, essentiellement à partir du rapport kinase/phosphatase.

Ainsi, les composition pharmaceutiques de l'invention sont particulièrement utiles pour la mise en oeuvre des tests de diagnostic des pathologies liées aux phénomènes d'angiogénèse, notamment pour le diagnostic des rétinopathies, de l'arthrite rhumatoïde, de la maladie de Crohn, de l'athérosclérose, l'hyperstimulation de l'ovaire, du psoriasis, de l'endométrie associée à la néovascularisation, de la resténose due à l'angioplastie du ballon, de la superproduction tissulaire due à la cicatrisation, de la maladie vasculaire périphérique, de l'hypertension, de l'inflammation vasculaire, de la maladie et les phénomènes de Raynaud, de l'anévrisme, de la resténose artérielle, de la thrombophlébite, de la lymphagyte, du lymphodème, de la cicatrisation et de la réparation tissulaire, de l'ischémie, de l'angine, de l'infarctus de myocarde, de la maladie chronique du coeur, des insuffisances cardiaques telles que l'insuffisance cardiaque congestive ou de la dégénération maculaire liée à l'âge et de l'ostéoporose.

D'autres avantages et caractéristiques de l'invention apparaîtront des exemples qui suivent dans lesquels on désigne par "oligonucléotide" l'oligonucléotide de séquence ID NO.3 et qui font référence aux figures jointes annexe dans desquelles
- la figure 1A représente l'image du Western Blot obtenu à partir des échantillons de surnageants issus des cellules non stimulées (Piste NS) et des cellules stimulées avec le bFGF (Piste S), révélée par un anticorps anti-IRS-1.
- la figure 1B montre l'image du Western blot obtenue après coloration au nitrate d'argent à partir des mêmes échantillons de surnageants issus des cellules non stimulées (Piste NS) et des cellules stimulées avec le bFGF (Piste S)
- la figure 2 montre les images du Western Blot obtenues à partir des échantillons des surnageants issus des cellules non stimulées (Piste NS) et des cellules stimulées avec le bFGF (Piste B), lorsque la membrane est incubée avec un anticorps monoclonal anti-phosphotyrosine et révélée avec un anticorps anti-isotype marqué à la peroxydase comme indiqué dans l'exemple 3,
- Lesfigures 3A à 3D montrent les images des cultures, sur un tapis du collagène type I, des différents lots de cellules endothéliales
- La figure 3A montre la culture des cellules endothéliales non traitées.
- La figure 3B montre la culture des cellules endothéliales stimulées avec 3 ng/ml de bFGF.
- lafigure 3C montre la culture des cellules endothéliales incubés avec 100 µg/ml d'oligonucléotide de séquence SEQ ID No. 3 pendant 4 heures, puis stimulées avec 3 ng/ml de bFGF.
- la figure 3D montre la culture des cellules endothéliales incubées avec 100 µg/ml de l'oligonucléotide de séquence SEQ ID No. 3 pendant 4 heures.
- Les figures 4A à 4F illustrent les résultats des essais d'inhibition de la néovascularisation cornéenne obtenue chez des rats.

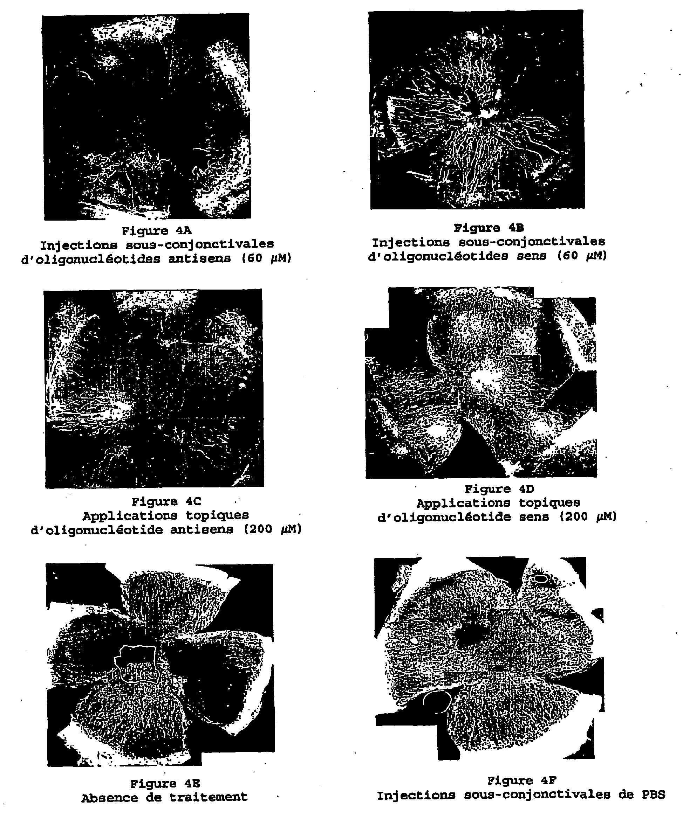

   La figure 4A montre les résultats obtenus par injection sous-conjonctivale d'un oligonucléotide antisens à une concentration de 60 µm.
   La figure 4B illustre les résultats obtenus après injection sous-conjonctivale d'un oligonucléotide sens à une concentration de 60 µM.
   La figure 4C montre les résultats obtenus après une application topique d'un oligonucléotide antisens à une concentration de 200µM.
   La figure 4D montre les résultats obtenues après une application topique d'un oligonucléotide sens à une concentration de 200µM.
   La figure 4E illustre l'état de la cornée en absence de tout traitement.
   La figure 4F montre l'état de la cornée lorsque celle-ci est traitée avec des injonctions sous-conjonctivales de PBS.

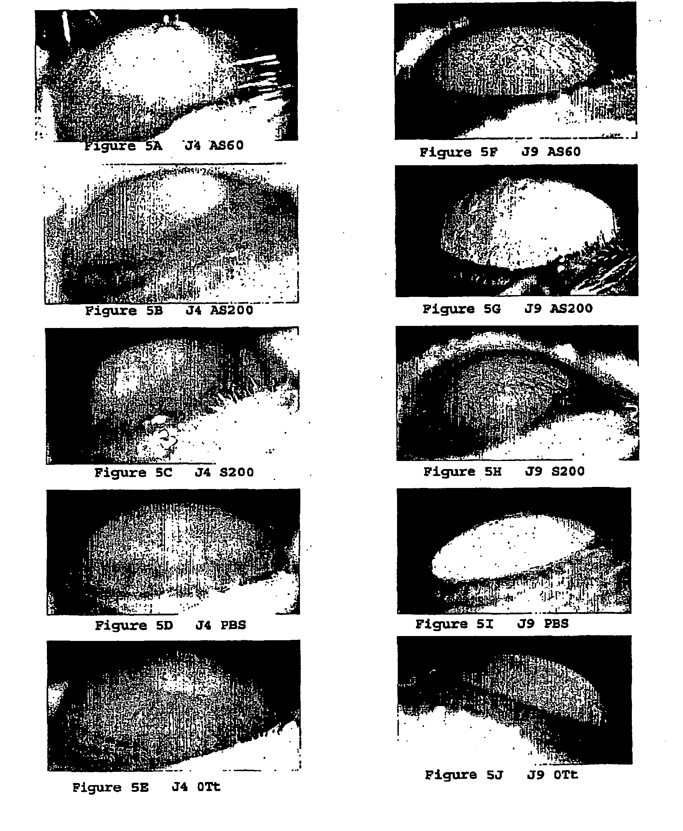

Les figures 5A à 5J illustrent les résultats de l'inhibition de la néovascularisation cornéenne obtenus chez les différents groupes de rats (décrits section 6.6 Résultats), après désépithélialisation et résection limbique des cornées de rats à jour 4 (Figures 5A à 5E) et à jour 9 (Figures 5F à 5J). Il s'agit de photographies en lampe à fente montrant la comparaison de la croissance des vaisseaux dans les différents groupes de rats. Grossissement x10

### Exemple 1 : Mise en évidence de l'induction de l'expression d'IRS-1 (la protéine de 180 kDa) dans les cellules endothéliales suite à la stimulation de ces cellules par le bFGF.

La protéine 180 kDa a été mise en évidence de la manière suivante :

Les cellules endothéliales ont été mises en culture dans une plaque de microtitration à 6 puits préalablement recouverts avec du collagène type **I** comme décrit dans (Montesano et al., J. Cell. Biol., 1983, 83, 1648-1652). Le milieu de culture est le DMEM (Sigma) enrichi avec 10 % de sérum de voeu fétal, 4 mM glutamine, 500 U/ml pénicilline, et 100 µg/ml streptomycine. Après 3 à 4 jours de culture, on obtient une couche semi-confluente des cellules endothéliales. Les milieux de culture de six puits ont été aspirés et remplacés par du milieu de culture frais. Trois puits ont été enrichis avec 3 ng/ml de bFGF. Après une incubation de 48 heures, les différents puits sont lavés trois fois avec un tampon phosphate, et les cellules sont utilisées pour extraire l'ARN messager (ARNm) selon des protocoles connus de l'homme du métier. Les ARNm sont reverse transcrits par une réaction de polymérisation en chaîne (PCR) en utilisant chacun des quatre groupes dégénérés d'amorces oligo(dT) (T12MN), M peut être G, A, ou C; et N est G, A, T, et C. Chaque groupe d'amorces est dicté par la base en position 3'(N), avec une dégénérescence dans la position (M). Exemple: le set d'amorces où N = G est constitué de:
SEQ ID No.24: 5'-TTTTTTTTTTTTGG-3'
SEQ ID No.25: 5'-TTTTTTTTTTTTAG-3'
SEQ ID No.26: 5'-TTTTTTTTTTTTCG-3'

Les ADNc ainsi obtenus sont amplifiés et marqués au moyen d'un décamère arbitraire, en présence d'ATP marqué isotopiquement. L'analyse des ADNc amplifiés par électrophorèse a révélé la présence d'un fragment d'ADNc de 326 bp amplifié dans l'échantillon issu des cellules endothéliales stimulées avec le bFGF, identifié dans la liste de séquences en annexe sous le numéro SEQ ID. No. : 27, cependant, ce même fragment est faiblement présent ou présent à l'état de trace dans l'échantillon issu des cellules endothéliales non stimulées avec le bFGF. Le séquençage dé ce fragment et l'interrogation subséquente des banques des données ont révélé que ce fragment correspond à une partie d'un gène déjà connu, codant pour le substrat du récepteur de l'insuline (une protéine intracellulaire de 180 kDa).

### Exemple 2 : Mise en évidence de l'induction de l'expression d'IRS-1 (la protéine de 180 kDa).

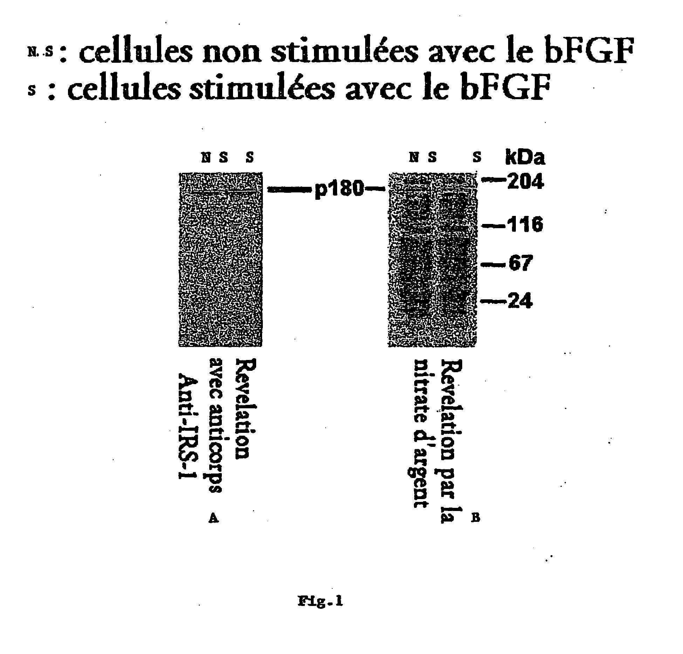

Des cellules endothéliales en culture sur un tapis du collagène type **I** stimulées ou non stimulées avec le bFGF (cf. exemple 1) sont lysées dans un tampon de lyse cellulaire contenant de l'orthovanadate de sodium. Ces solutions sont ensuite clarifiées par centrifugation à 14000 g pendant 15 min. Des échantillons de surnageants issus des cellules non stimulées et des cellules stimulées avec le bFGF, contenant des quantités équivalentes de protéines, ont été repris avec une solution d'électrophorèse contenant 2% SDS et 15 mM de dithiothréitol, chauffés à 100°C pendant 5 min. puis déposés en gel de polyacrylamide (gradient de 4 à 15% en acrylamide) dans des conditions dénaturantes (en présence de 2% SDS). Après migration, les protéines sont transférées sur une membrane de nitrocellulose. La membrane est bloquée par une incubation à température ambiante dans une solution de 5% de lait dans un tampon PBS. La membrane est ensuite lavée trois fois avec un tampon PBS, incubée dans un tampon PBS contenant 1 µg/ml d'anticorps monoclonal anti-IRS-1 pendant 2 heures à température ambiante, et lavée trois fois avec un tampon PBS. Les protéines sont ensuite révélées au moyen d'un anticorps secondaire anti-isotype couplé à la peroxydase. On constate la présence d'une protéine de poids moléculaire 180 kDa reconnue par l'anticorps anti-IRS-1 monoclonal dans les préparations issues des cellules endothéliales stimulées avec le bFGF; cette protéine est faiblement présente dans la préparation issue des cellules endothéliales non stimulées avec le bFGF (Figure 1).

### Exemple 3 : Mise en évidence de l'induction de la phosphorylation au niveau de tyrosine d'IRS-1 (la protéine de 180 kDa).

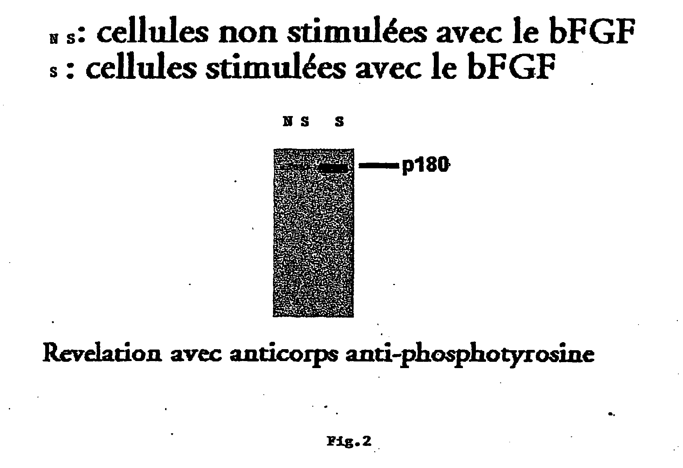

Des cellules endothéliales humaines en culture sur un tapis du collagène type **I** stimulées ou non stimulées avec le bFGF sont lysées dans un tampon de lyse cellulaire contenant de l'orthovanadate de sodium. Ces solutions sont ensuite clarifiées par centrifugation à 14000 g pendant 15 min (cf. exemple 2). La protéine IRS-1 a été extraite grâce à un anticorps monoclonal anti-IRS-1. Cette extraction est réalisée après une immunoprécipitation à l'aide d'un anticorps monoclonal anti-IRS-1 (Sigma). Après addition de l'anticorps anti-IRS-1 couplé à l'agarose, la suspension est incubée pendant 2 heures à température ambiante, puis centrifugée à 4000 g pendant 15 min. Le précipité obtenu est repris avec une solution d'électrophorèse contenant 2% SDS et 15 mM de dithiothréitol, chauffé à 100°C pendant 5 min., puis déposé en gel de polyacrylamide (gradient de 4 à 15% en acrylamide) dans des conditions dénaturantes (en présence de 2% SDS). Après migration, les protéines sont transférées sur une membrane de nitrocellulose. La membrane est bloquée par une incubation à température ambiante dans une solution de 5% de lait dans un tampon PBS. La membrane est ensuite lavée trois fois avec un tampon PBS, incubée dans un tampon PBS contenant 1 µg/ml d'anticorps monoclonal anti-phosphotyrosine pendant 2 heures à température ambiante, et lavée trois fois avec un tampon PBS. Les protéines sont ensuite révélées au moyen d'un anticorps secondaire anti-isotype couplé à la peroxydase. On constate que la protéine IRS-1 de poids moléculaire 180 kDa est phosphorylée au niveau du résidu tyrosine dans les préparations issues des cellules endothéliales stimulées avec le bFGF ; cette protéine est très faiblement phosphorylée au niveau du résidu tyrosine dans la préparation issue des cellules endothéliales non stimulées avec le bFGF (Figure 2)

### Exemple 4 : Evaluation de l'activité aniti-angiogénique in vitro de l'oligonucléotide.

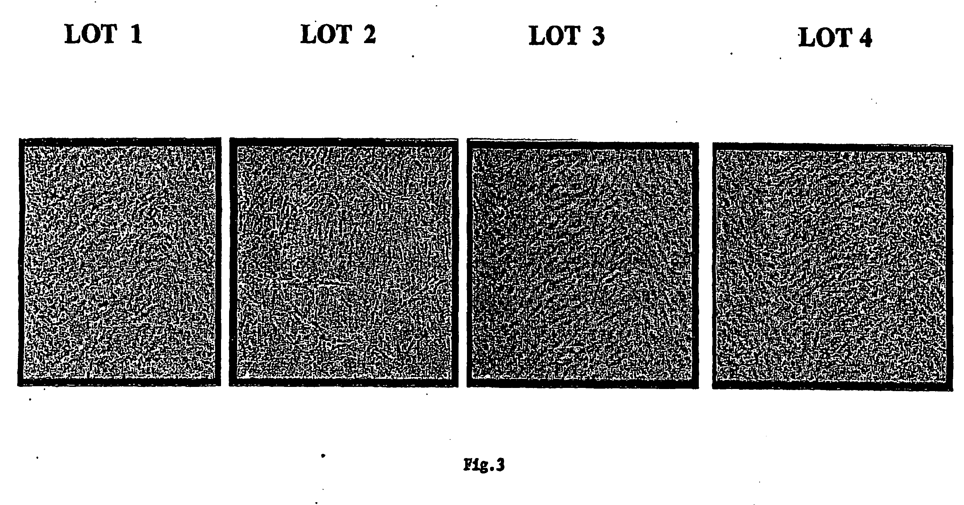

Des cellules endothéliales humaines sont mises en culture sur un tapis du collagène type I. Au septième jour de culture, les puits de culture sont divisés en quatre lots :
Lot 1 : Puits correspondant à la culture des cellules endothéliales non traitées. (Figure 3A)
Lot 2 : Puits correspondant à la culture des cellules endothéliales stimulées avec 3 ng/ml de bFGF. (Figure 3B)
Lot 3 : Puits correspondant à la culture des cellules endothéliales incubées avec 100 µg/ml d'oligonucléotide de séquence SEQ ID No. 3 pendant 4 heures, puis stimulées avec 3 ng/ml de bFGF. (Figure 3C)
Lot 4 : Puits correspondant à la culture des cellules endothéliales incubées avec 100 µg/ml d'oligonucléotide de séquence SEQ ID No. 3 pendant 4 heures. (Figure 3D)

Après 3 à 4 jours de culture, les différents puits sont examinés à l'aide de microscope optique à phase inverse. A la lecture des résultats, il apparaît que les cellules endothéliales humaines dans le lot 2 forment des tubes capillaires suite à la stimulation avec le bFGF. On constate aussi que l'oligonucléotide inhibe la formation de néovaisseaux par ces mêmes cellules stimulées avec le bFGF dans le lot 3. On observe enfin que l'oligonucléotide ne modifie pas de manière prononcée la croissance des cellules endothéliales. En effet, le nombre des cellules endothéliales dans le puits de lot 1 et ceux de lot 4 sont comparables.

### Exemple 5 : Evaluation de l'activité in vivo de l'oligonucléotide.

Trois lots de souris nude ont été utilisés.
Chaque lot étant constitué de 5 souris.
Lot N°1 : Ce lot a servi de témoin. Chaque souris est inoculée en sous-cutané à J0 avec 200 µl d'une suspension de cellules de mélanome B16 (fournies par l'Institut Gustave Roussy de Villejuif) dispersées dans du PBS à raison de 10⁶ cellules/ml. Ces souris ne sont pas traitées par la suite.
Lot N°2 . Chaque souris est inoculée en sous-cutané à J0 avec 200 µl d'une suspension de cellules de mélanome B16 dispersées dans du PBS à raison de 10⁶ cellules/ml. A J1, J2, J3, J4, J5, J6, J7, J8, J9 et J10 chaque souris reçoit une injection en sous-cutanée de 200 µl d'une solution d'oligonucléotide diluée dans du PBS à une concentration de 500 mg/ml. L'injection d'oligonucléotide est effectuée à proximité du site d'injection des cellules.
Lot N°3 : Les souris de ce lot ne sont pas inoculées avec les cellules de mélanome B16. Cependant, chacune de ces souris reçoit une injection de 200 µl d'une solution d'oligonucléotide dans du PBS à une concentration de 500 µg/ml les injections sont faites à J1, J2 J3, J4, J5, J6, J7, J8, J9 et J10.

Les résultats sont les suivants :

Après inoculation, chez les souris du lot N°1, la masse tumorale se développe d'une façon très rapide. En effet, la masse tumorale atteint une taille de 1,6 à 2,5 cm de diamètre après dix jours chez les souris dudit lot N°1 (souris non traitées). L'évolution de la masse tumorale chez les souris du lot N°2 (souris traitées après inoculation par injection d'oligonucléotide à J1, J2 et J3), montre une augmentation nettement moindre du volume de la masse tumorale. La masse tumorale chez les souris du lot 2 ne dépasse pas 0,8 cm de diamètre au dixième jour. Au quatorzième jour, la différence entre la masse tumorale des souris de lots N°2 et celle des souris du lot N°1 est remarquable.

Chez les souris du lot N°3 (souris n'ayant pas reçu de cellules de mélanome B16, mais traitées par injection d'oligonucléotide pendant trois jours) on note un effet général inattendu sur la peau. Il est identique à celui noté chez toutes les souris qui ont été traitées par l'oligonucléotide (lot 2). La peau prend un aspect vieilli, fripé. On note aussi l'apparition de poils chez toutes les souris traitées. Il existe un parallélisme au cours de l'évolution entre la régression des signes cutanés et la reprise de la croissance tumorale.

On constate donc que l'oligonucléotide inhibe le développement et la formation des néovaisseaux par les cellules endothéliales *in vitro*; Il possède d'autre part une activité anti-tumorale *in vivo* remarquable chez la souris nude.

### Exemple 6: Evaluation de l'oligonucléotide anti-angiogénique sur un modèle de néovascularisation cornéenne chez le rat

Sur la base des travaux d'Amano et al. (1998), le demandeur a repris, modifié et analysé un modèle de formation de néovaisseaux cornéens chez le rat après désépithélialisation et limbectomie (Figures 5A à 5J). Il est reproductible, permet d'examiner directement les néovaisseaux en lampe à fente et de les quantifier, les détails sont indiqués ci-dessous. Ce modèle a ensuite été utilisé pour tester l'efficacité d'agents anti-angiogéniques de l'invention.

### 6.1 Animaux et modèle de néovascularisation cornéenne.

Des rats (Rattus norvegicus) Wistar mâles âgés de cinq semaines (Charles River France, St-Aubin les Elbeufs, France), exempts de germes pathogènes spécifiques sont nourris et abreuvés à volonté, et maintenus, dans l'animalerie du laboratoire, dans des conditions fixes de température et d'humidité, avec des cycles de 12 heures de lumière / 12 heures d'obscurité.

Les rats sont anesthésiés avec un mélange de kétamine (Kétamine 1000, UVA, Ivry-sur-Seine, France ; 128 mg/kg) et de chlorpromazine (Largactil 25 mg/ml; Specia Rhône Poulenc, Paris, France; 5 mg/kg), injecté par voie intra-musculaire. Une goutte d'oxybuprocaïne (Novésine, Chibret, Clermont-Ferrand, France) est instillée dans l'oeil droit. A l'aide d'un système grossissant (macroscope Wild MPS 51 S, LEICA, Heerbrugg, Suisse), l'épithélium cornéen est enlevé par une « microsponge » imbibée d'éthanol à 70%. Une bande de conjonctive de 1,5 mm de large, au limbe, est excisée à l'aide de ciseaux microchirurgicaux, et les paupières sont fermées par une blépharorraphie temporaire à l'aide d'un fil Vicryl 5.0 (Dacron, Alcon, Rueil-Malmaison, France). L'oeil est ensuite rincé abondamment au PBS 1X, une pommade xytétracycline (Posicycline, Alcon, France) est appliquée et la blépharorraphie est ouverte le quatrième jour (8,9).

### 6.2. Traitement par injections sous-conjonctivales et applications topiques de l'oligonucléotide anti-angiocrénique.

L'ensemble de rats a été divisé en 6 groupes :
Groupe A : modèle + injection sous-conjonctivale d'une solution d'oligonucléotide antisens à 60 µM dans du PBS 1X,
Groupe B : modèle + application topique d'une solution d'oligonucléotide antisens à 200 µM dans du PBS 1X,
Groupe C : modèle + injection sous-conjonctivale d'une solution d'oligonucléotide sens à 60 µM dans du PBS 1X,
Groupe D : modèle + application topique d'une solution d'oligonucléotide sens à 200 µM dans du PBS 1X,
Groupe E : modèle + injection sous-conjonctivale de PBS 1X,
Groupe F : modèle sans traitement).

Tous les rats ont été désépithélialisés comme décrit ci-dessus ; le traitement a été effectué toutes les 24 heures à partir du quatrième jour, et jusqu'au neuvième jour. La néovascularisation a été examinée en début, milieu et fin de protocole, par examen en lampe à fente ; des photographies ont été prises à J0 et J9.

### 6.3 Visualisation et quantification de la néovascularisation.

Les animaux ont été euthanasiés 10 jours après la désépithélialisation, par injection létale de pentobarbital (injection intra-péritonéale). Pour remplir les micro-vaisseaux et quantifier la néovascularisation cornéenne, la partie supérieure du corps de l'animal a été perfusée avec de la Fluorescéine-Dextran 2x1 000 000. Les yeux ont été énucléés et immergés dans du paraformaldéhyde/PBS 1X 4% pendant 3 heures, puis une nuit dans du PBS 1X. On a ensuite isolé la cornée avec 1 mm de limbe, sous microscope opératoire, et on l'a mise à plat entre lame et lamelle grâce à 3 à 5 incisions radiaires. Les cornées à plat ont ensuite été examinées et photographiées en microscopie à fluorescence. Une fois les cornées entières reconstituées, on les a scannées puis on a mesuré les surfaces par analyse d'image ; un logiciel (NIH image) permettant la quantification de la néovascularisation. Pour chaque photo, on a mesuré 3 fois la surface cornéenne totale, et 3 fois la surface néovascularisée ; le rapport des moyennes - surface néovascularisée / surface cornéenne totale - nous permet d'obtenir le « pourcentage de néovascularisation », et de mesurer l'inhibition obtenue.

### 6.4 Analyste statistique

Les résultats sont exprimés en moyennes ± SD. Les pourcentages de surface néovascularisée/ surface totale ont été comparés avec le test non paramétrique de Mann-Whitney. Les valeurs de P<0,05 sont considérées comme significatives.

### 6.5 Dilution de l'oligonucléotide

L'oligonucléotide est dilué dans du PBS 1X à pH 7,2. En fonction des données de la littérature et des expériences menées par ailleurs avec d'autres oligonucléotides, il a été choisi d'utiliser une concentration de 60 µM pour les injections sous-conjonctivales, et une concentration de 200 µM pour les applications topiques.

### 6.6 Résultats

Sur le modèle de néovaisseaux cornéens, il a été effectué un traitement avec l'oligonucléotide 5'-TATCCGGAGGGCTCGCCATGCTGCT-3', identifié sous le numéro SEQ IQ No. 3 dans la liste de séquences en annexe, modifié sous forme phosphorothioate, par jour, de J4 à J9, selon le protocole suivant :
Groupe A : injection sous-conjonctivale de l'oligonucléotide antisens à 60 µM (AS 60)
Groupe B : application topique de l'oligonucléotide antisens à 200 µM (AS 200)
Groupe C : injection sous-conjonctivale de l'oligonucléotide sens à 60 µM (S 60)
Groupe D : application topique de l'oligonucléotide sens à 200 µM (S 200)
Groupe E : injection sous-conjonctivale de PBS 1X (PBS)
Groupe F : pas de traitement (O Tt)

Le dixième jour du protocole, les rats ont été perfusés avec une solution de FITC/Dextran, puis euthanasiés, les cornées ont été prélevées et fixées dans une solution de PAF 4%; ensuite les cornées ont été mises à plat entre lame et lamelle dans une solution de glycérol; les néovaisseaux fluorescents ont été observés et photographiés au microscope à fluorescence, les photos ont été scannées, et mesuré les pourcentages de néovascularisation pour chaque animal.

Les résultats observés sont présentés dans le tableau 1 ci-dessous :

**Tableau 1**

| | **Groupe A** | **Groupe B** | **Groupe C** | **Groupe D** | **Groupe E** | **Groupe F** |
|---|---|---|---|---|---|---|
| | **AS 60** | **AS 200** | **S 60** | **S 200** | **PBS** | **O Tt** |
| Moyenne | 0,6157 | 0,5058 | 0,9431 | 0,9392 | 0,9552 | 0,9170 |
| SD | 0,2194 | 0,1172 | 0,0964 | 0,0308 | 0,0481 | 0,0751 |
| Nombre de mesures | 15 | 15 | 15 | 12 | 9 | 9 |
| SEM | 0,0566 | 0,0303 | 0,0249 | 0,0089 | 0,0160 | 0,0250 |

L'analyse statistique des résultats par un test non paramétrique de Mann-Whitney donne les résultats suivantes :

Les injections sous-conjonctivales d'ODN antisens à 60 µM (A) ont réduit la néovascularisation par rapport aux groupes E et F, témoins (résultats très significatifs, P<0,0001 et P=0,0011) ; l'application topique de l'oligonucléotide antisens à une concentration de 200 µM (B) a réduit la néovascularisation par rapport aux groupes témoins E et F (résultats extrêmement significatifs, P<0,0001).

Comparées à l'administration sous-conjonctivale de d'ODN sens à 60 µM (C) ou à l'application topique d'ODN sens à 200 µM (D), l'injection d'ODN antisens à 60 µM (A) et l'application topique d'ODN antisens à 200 µM (B) ont réduit la néovascularisation. Ces résultats sont extrêmement significatifs P<0,0001. (Figures 4A à 4F).

L'inhibition de la néovascularisation n'est pas significativement différente, selon que l'on administre l'oligonucléotide antisens par voie sous-conjonctivale (60 µM) ou par voie topique (200 µM) : elle est d'environ 35% par rapports aux témoins (E et F).

L'injection sous-conjonctivale d'ODN sens à 60 µM (C) et l'application topique d'ODN sens à une concentration de 200 µM (D) n'ont pas modifié la néovascularisation par rapport aux groupes témoins (E et F). Par contre, on observe un petit effet de l'oligonucléotide sens en application topique (D) par rapport à l'oligonucléotide sens en injections sous-conjonctivales (C) (P=0,0117).

Par ailleurs, on observe, dans les groupes traités par l'antisens (A et B), un calibre et une densité moindres des néovaisseaux. Leur répartition n'est pas différente par rapport aux groupes témoins, et aucune différence de niveau d'inflammation n'est observée (Figure 4).

### 6.7 Effets secondaires

Au cours des deux séries d'expériences, aucun effet secondaire notable n'a été observé dans aucun groupe : après 6 jours de traitement aux doses mentionnées ci-dessus, la peau des rats n'était pas fripée, le pelage ne s'est pas modifié, l'état général des animaux était bon ; ils se sont nourris normalement jusqu'au dernier jour, et aucune mortalité suspecte n'a été observée. Bien qu'il n'ait été pratiqué ni autopsie, ni analyse sanguine, l'état général des animaux en fin d'expérimentation ne laisse pas supposer l'existence de troubles hépatiques. Le seul symptôme observé est un dépôt blanchâtre transitoire, à l'endroit des injections sous-conjonctivales, chez 60% des rats du groupe A, 60% des rats du groupe C, et 10% des rats du groupe E. Ce dépôt s'est résorbé en fin d'expérimentation dans tous les cas.

Cet exemple montre que, contrairement à ce qu'on pouvait attendre, les injections sous-conjonctivales d'oligonucléotide antisens à une concentration de 60µM n'ont pas plus inhibé la néovascularisation que l'administration topique d'oligonucléotide antisens à une concentration de 200µM.

Ceci peut s'expliquer en partie par la différence des concentrations utilisées ; mais ce résultat suggère également une pénétration de l'oligonucléotide par voie topique plutôt que par le limbe. Il suggère également l'absence de libération prolongée du produit à partir du site d'injection.

### 6.8 Conclusion

L'application de l'oligonucléotide antisens, par voie topique ou en injections sous-conjonctivales réduit la néovascularisation sur notre modèle de néovaisseaux cornéens chez le rat.
Le but de ce travail était de tester l'efficacité des oligonucléotides anti-sens issus de la séquence du gène IRS-1 sur un modèle de néovascularisation cornéenne chez le rat préalablement mis au point.
Ce modèle est facilement accessible, reproductible, et quantifiable. De plus, cette étude a permis une première évaluation de la concentration d'oligonucléotide nécessaire, *in vivo,* pour inhiber une néovascularisation.

### BIBLIOGRAPHIE

1-Aiello LP. Keeping in touch with angiogenesis. Nat Med 2000 ; 6 : 379-381
2-D'Amore P.A. Mechanisms of Retinal and Choroidal Neovascularization. Invest Ophthalmol Vis Sci 1994; 35(12) : 3974-3979
3-Hélène C. Rational design of sequence-specific oncogene inhibitors based on antisense and antigène oligonucleotides. Eur J Cancer 1991 ; 27 : 1466-1471
4-Agrawal S, Bunnel BA, Crooke ST, Davidkova G, Gyurko R, Iyer K et al. Antisense oligonucleotides and antisense RNA. Benjamin Weiss edition (Philadelphie, USA) 1997 ; 1-11, 19-40
5-Pierga JY, Cammilleri S, Benyahia B, Magdelénat H. Applications des oligonucléotides antisens en cancérologie. Bull Cancer 1994 ; 81 : 1023-1042
6-Robinson GS, Pierce EA, Rook SL, Foley E, Webb R, Smith LEH. Oligodeoxynucleotides inhibit retinal neovascularization in a murine model of proliferative retinopathy. Proc Natl Acad Sci USA 1996 ; 93 : 4851-4856
7-Aiello LP. Vascular endothelial growth factor. Invest ophthalmol Vis Sci 1997 ; 38 :1647-1652
8-Amano S, Rohan R, Kuroki M, Tolentino M, Adamis AP. Requirement for vascular endothelial growth factor in wound and inflammation-related corneal neovascularization. Invest Ophthalmol Vis Sci 1998 ; 39 : 18-22
9-Hoang-Xuan T, Prisant O. Restauration de l'épithélium cornéen à partir des cellules souches limbiques. Med Sci 1998 ; 14 : 1375-1377
10-Parry T.J, Cushman C, Gallegos A.M, Agrawal A.B, Richardson M, Andrews L.E. et al. Bioactivity of antiangiogenic ribozymes targeting Flt-1 and KDR mRNA. Nucleic Acids Research 1999; 27: 2569-2577
11-ozaki H, Seo MS, Ozaki K, Yamada H, Yamada E, Okamoto N et al. Blockade of vascular endothelial cell growth factor receptor signaling is sufficient to completely prevent retinal neovascularization. Am J Pathol 2000; 156: 697-707
12-Berdugo Polak M, Administration par iontophorèse d'oligonucleotides antisens dans le segment antérieur de l'aeil : application à un modèle, de néovascularisation cornéenne chez le rat. DEA "Biologie et Pathologie des Epithéliums"; université Paris VII, Feldmann G.; Inserm U450, directeur Courtois Y., sous la direction de Behar Cohen F. 2000

## Revendications

1. Composition pharmaceutique comprenant, à titre d'agent actif, la séquence SEQ ID No. 3 ou un fragment de ladite séquence comprenant au moins douze nucléotides contigus.

2. Composition pharmaceutique selon la revendication 1 dans laquelle l'agent actif est associé dans ladite composition avec un véhicule acceptable choisi parmi les suspensions aqueuses, les solutions salines isotoniques et les solutions stériles injectables.

3. Composition pharmaceutique selon l'une quelconque des revendications 1 ou 2, **caractérisée en ce qu'**elle se présente sous une forme adaptée à une administration par voie sous-cutanée, intramusculaire, intraveineuse ou transdermique.

4. Composition pharmaceutique selon l'une quelconque des revendications 1 à 3, **caractérisée en ce qu'**elle comprend de 0,001 mg à 50 mg d'agent actif.

5. Utilisation de la séquence SEQ ID No. 3 ou d'un fragment de ladite séquence comprenant au moins douze nucléotides contigus à titre d'agent actif pour la fabrication d'un médicament utile pour le traitement des pathologies liées l'angiogénèse.

6. Utilisation selon, la revendication 5 de la séquence SEQ ID No. 3 ou d'un fragment de ladite séquence d'au moins douze nucléotides contigus à titre d'agent actif pour la fabrication d'un médicament utile pour le traitement des maladies de l'oeil liées à la néovascularisation,

7. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement de la néovascularisation cornéenne.

8. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement des rétinopathies.

9. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement de la rétinopathie diabétique.

10. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement de la dégénérescence maculaire liée à l'âge (DMLA).

11. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement du psoriasis.

12. Utilisation selon la revendication 5 de .la séquence SEQ ID No. 3 ou d'un fragment de ladite séquence d'au moins douze nucléotides contigus à titre d'agent actif pour la fabrication d'un médicament utile pour le traitement de la superproduction tissulaire due à la cicatrisation.

13. Utilisation selon la revendication 5 de la séquence SEQ ID No. 3 ou d'un fragment de ladite séquence d'au moins douze nucléotides contigus à titre d'agent actif pour la fabrication d'un médicament utile pour le traitement des tumeurs.

14. Utilisation selon l'une quelconque des revendications 5 à 13 dans laquelle ledit agent actif est associé dans ledit médicament avec un véhicule acceptable choisi parmi les suspensions aqueuses, les solutions salines isotoniques ou les solutions stériles injectables.

15. Utilisation selon l'une quelconque des revendications 5 à 14, **caractérisée en ce que** ledit médicament se présente sous une forme pouvant être administrée par voie sous-cutanée, intramusculaire, intraveineuse ou transdermique.

16. Utilisation selon l'une quelconque des revendications 5 à 15, **caractérisée en ce que** le médicament comprend de 0,001 mg à 50 mg d'agent actif.

## Claims

1. A pharmaceutical composition comprising, as active agent, the sequence SEQ ID NO 3 or a fragment of said sequence comprising at least twelve contiguous nucleotides.

2. The pharmaceutical composition according to claim **1,** wherein said active agent is associated in said composition to an acceptable vehicle selected among aqueous suspensions, isotonic saline solutions and injectable sterile solutions.

3. The pharmaceutical composition according to any one of claim **1** or **2,** wherein the composition is in a form capable of subcutaneous, intramuscular, intravenous or transdermal administration.

4. The pharmaceutical composition according to any one of claim **1** to **3,** wherein the composition comprises 0.001 mg to 50 mg of active agent.

5. Use of the sequence SEQ ID NO: 3 or a fragment of said sequence comprising at least twelve contiguous nucleotides as active agent for the preparation of a medicament useful for treating angiogenesis-linked pathologies.

6. The use according to claim **5** of the sequence SEQ ID NO: 3 or of a fragment of said sequence comprising at least twelve contiguous nucleotides for the preparation of a medicament useful for treating eye diseases linked to neovascularisation.

7. The use according to claim **5** of the sequence SEQ ID NO: 3 or of a fragment of said sequence comprising at least twelve contiguous nucleotides for the preparation of a medicament useful for treating corneal neovascularisation.

8. The use according to claim **5** of the sequence SEQ ID NO: 3 or of a fragment of said sequence comprising at least twelve contiguous nucleotides for the preparation of a medicament useful for treating retinopathies.

9. The use according to claim **5** of the sequence SEQ ID NO: 3 or of a fragment of said sequence comprising at least twelve contiguous nucleotides for the preparation of a medicament useful for treating diabetic retinopathies.

10. The use according to claim **5** of the sequence SEQ ID NO: 3 or of a fragment of said sequence comprising at least twelve contiguous nucleotides for the preparation of a medicament useful for treating age-related macular degeneration (DMLA).

11. The use according to claim **5** of the sequence SEQ ID NO: 3 or of a fragment of said sequence comprising at least twelve contiguous nucleotides for the preparation of a medicament useful for treating psoriasis.

12. The use according to claim **5** of the sequence SEQ ID NO: 3 or of a fragment of said sequence comprising at least twelve contiguous nucleotides for the preparation of a medicament useful for treating tissue superproduction due to cicatrization.

13. The use according to claim **5** of the sequence SEQ ID NO: 3 or of a fragment of said sequence comprising at least twelve contiguous nucleotides for the preparation of a medicament useful for treating tumors.

14. The use according to any one of claims **5** to **13,** wherein said active agent is associated in said medicament to an acceptable vehicle selected among aqueous suspensions, isotonic saline solutions and injectable sterile solutions.

15. The use according to any one of claims **5** to **14,** wherein said medicament is in a form capable of subcutaneous, intramuscular, intravenous or transdermal administration.

16. The use according to any one of claims **5** to **15,** wherein said medicament comprises 0.001 mg to 50 mg of active agent.

## Patentansprüche

1. Pharmazeutische Zusammensetzung, welche als Wirkstoff die Sequenz SEQ ID Nr. 3 oder ein mindestens zwölf aneinandergereihte Nukleotide aufweisendes Fragment dieser Sequenz enthält.

2. Pharmazeutische Zusammensetzung nach dem Anspruch 1, in der der Wirkstoff in der besagten Zusammensetzung mit einem geeigneten, aus wässrigen Lösungen, isotonischen salzigen Lösungen und injizierbaren sterilen Lösungen ausgewählten Trägerstoff verbunden ist.

3. Pharmazeutische Zusammensetzung nach einem der Ansprüche 1 oder 2, **dadurch gekennzeichnet, dass** sie sich in einer für eine subkutane, intramuskuläre, intravenöse oder transdermale Verabreichung geeigneten Form darstellt.

4. Pharmazeutische Verbindung nach einem der Ansprüche 1 bis 3, **dadurch gekennzeichnet, dass** sie 0,001 mg bis 50 mg Wirkstoff enthält.

5. Verwendung der Sequenz SEQ ID Nr. 3 oder eines mindestens zwölf aneinandergereihte Nukleotide aufweisenden Fragments dieser Sequenz als Wirkstoff zur Herstellung eines Medikaments, das für die Behandlung von mit einer Angiogenese verbundenen Symptomatiken nutzbar ist.

6. Verwendung der Sequenz SEQ ID Nr. 3 nach Anspruch 5 oder eines mindestens zwölf aneinandergereihte Nukleotide aufweisenden Fragments dieser Sequenz als Wirkstoff nach dem Anspruch 5 zur Herstellung eines Medikaments, das für die Behandlung von mit einer Neovaskularisation verbundenen Augenkrankheiten nutzbar ist.

7. Verwendung der Sequenz SEQ ID Nr. 3 nach Anspruch 5 oder eines mindestens zwölf aneinandergereihte Nukleotide aufweisenden Fragments dieser Sequenz als Wirkstoff nach dem Anspruch 5 zur Herstellung eines Medikaments, das für die Behandlung einer Hornhaut-Neovaskularisation nutzbar ist.

8. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung einer Retinopathie nutzbar ist.

9. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung einer diabetischen Retinopathie nutzbar ist.

10. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung einer altersbedingten Makuladegeneration (AMD) nutzbar ist.

11. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung einer Psoriasis nutzbar ist.

12. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung einer Gewebewucherung aufgrund einer Narbenbildung nutzbar ist.

13. Verwendung der Sequenz SEQ ID Nr. 3 nach Anspruch 5 oder eines mindestens zwölf aneinandergereihte Nukleotide aufweisenden Fragments dieser Sequenz als Wirkstoff zur Herstellung eines Medikaments, das für die Behandlung von Tumoren nutzbar ist.

14. Verwendung nach einem der Ansprüche 5 bis 13, in der der Wirkstoff in dem Medikament mit einem geeigneten, aus wässrigen Lösungen, isotonischen salzigen Lösungen oder injizierbaren sterilen Lösungen ausgewählten Trägerstoff verbunden ist.

15. Verwendung nach einem der Ansprüche 5 bis 14, **dadurch gekennzeichnet, dass** sich das Medikament in einer Form darstellt, in der es subkutan, intramuskulär, intravenös oder transdermal verabreicht werden kann.

16. Verwendung nach einem der Ansprüche 5 bis 15, **dadurch gekennzeichnet, dass** das Medikament 0,001 mg bis 50 mg Wirkstoff aufweist.
